# EUROPÄISCHE PATENTSCHRIFT

(11) **EP 0 473 064 B1**
(45) Veröffentlichungstag und Bekanntmachung des Hinweises auf die Patenterteilung: **14.06.1995**
(21) Anmeldenummer: 91114071.3
(22) Anmeldetag: 22.08.1991
(51) Int. Cl.: D04H 1/00

(54) **Verfahren zur Herstellung von Füllmaterial für dreidimensional geformte textile Gebilde sowie Vorrichtung dafür**
Process for making a filling material for three-dimensional textile structure and apparatus therefor
Procédé pour la fabrication de matériau de remplissage pour enveloppe textile tridimensionnelle et son installation

(30) Priorität: 25.08.1990 DE 4026916
(43) Veröffentlichungstag der Anmeldung: 04.03.1992
(73) Patentinhaber: HOECHST AKTIENGESELLSCHAFT, 65926 Frankfurt am Main (DE)
(72) Erfinder: Nettelnstroth, Karl, W-8900 Augsburg (DE)

(56) Entgegenhaltungen:
- EP-A- 0 313 839
- EP-A- 0 371 807
- DE-A- 3 804 222
- GB-A- 1 280 818

## Beschreibung

Die vorliegende Erfindung betrifft ein neues Herstellungsverfahren für ein textiles Füllmaterial für dreidimensional geformte textile Gebilde, sowie eine angepaßte Vorrichtung zur Durchführung des Verfahrens.

Als Füllmaterial für dreidimensional geformte textile Gebilde, wie beispielsweise Kissen oder Bettwarenartikel werden üblicherweise Federn und/oder Daunen und/oder Fasern verwendet. Die Füllfasern werden dabei in der Regel als Krempelvliese eingebracht. Diese Gebilde sind mechanisch nicht sehr stabil, da die Fasern nur locker aneinander haften. Bei der Benutzung reißen diese Vliese daher häufig auseinander und es bilden sich Klumpen, die nicht mehr zu trennen sind. Derartig verfestigte Füllvliese auf Basis von Bikomponentenfasern sind aus der US-A-3,589,956 bekannt.

Es ist bereits versucht worden, Wattetupfer aus Wattebändern herzustellen, indem man ein Watteband aus saugfähigen Fasern und zumindest oberflächig schmelzbaren Fasern durch Hitzebehandlung verfestigt und die Wattetupfer anschließend aus dem verfestigten Watteband ausstanzt. So wird in der DE-A-3,804,222 ein solches Verfahren beschrieben, das dadurch gekennzeichnet ist, daß das Watteband an den auszustanzenden Stellen selektiv erwärmt und anschließend gekühlt wird, bevor das Ausstanzen erfolgt. Bei den vorbekannten Verfahren ist mit erheblichen Abfallverlusten zu rechnen, da durch das Ausstanzen aus dem Band nur Teile davon direkt verwendet werden können. Nach den Angaben in der DE-A-3,804,222 kann der Abfall etwa 35 bis 45 % des Wattebandes betragen.

Aus der GB-A-1,280,818 ist ein Verfahren zur Herstellung von Zigarettenfiltern bekannt, bei dem ein Kabel aus Heterofilamenten zum Einsatz kommt.

Es wurde jetzt ein Verfahren zur Herstellung von Füllmaterial für dreidimensional geformte textile Gebilde gefunden, in dem praktisch keine Verluste durch Verschnitt auftreten und mit dem ein Füllmaterial hergestellt werden kann, das nicht zur Klumpenbildung neigt und das sich aufschütteln läßt. Mit dem nach dem erfindungsgemäßen Verfahren hergestellten Füllmaterial kann ein stabilisiertes Volumen ausgebildet werden, das auch nach längerer Benutzungsdauer durch Aufschütteln erhalten bleibt.

Das Verfahren ist gekennzeichnet durch die in Anspruch 1 definierten Maßnahmen.

Die Begriffe "kontinuierliches Führen des Faserkabels" und "kontinuierliches Zuführen zu einer Schneidevorrichtung" bedeuten, daß das Faserkabel zumindest von der Erhitzungszone bis zur Schneidevorrichtung ohne Unterbrechung bewegt werden soll.

Die verschiedenen Zonen im erfindungsgemäßen Verfahren können horizontal oder auch vertikal angeordnet sein. Bei vertikaler Anordnung kann die Zuführzone sich am oberen oder auch am unteren Teil der Vorrichtung befinden. Befindet sich diese im oberen Teil der Vorrichtung, so wird das zu verarbeitende Faserkabel zunächst nach oben geführt und in die Zuführzone eingeführt und bewegt sich dann nach unten durch die Erhitzungs- und die Abkühlungszone auf die Schneidezone zu. Befindet sich die Zuführzone am unteren Teil der Vorrichtung, so bewegt sich das zu verarbeitende Faserkabel durch die Erhitzungs- und Abkühlungszone nach oben auf die Schneidezone zu.

Im erfindungsgemäßen Verfahren läßt sich praktisch jedes Faserkabel einsetzen, sofern es aus einer Kombination von Füllfasern und Bindefasern besteht. Dabei kann es sich um Faserkabel aus Endlosfasern handeln oder insbesondere um Faserkabel aus Stapelfasern. Die Faserkabel aus Stapelfasern können dabei auf beliebige Weise hergestellt werden. So kann es sich dabei beispielsweise um Reißkabel, Kardenbänder oder Krempellunten handeln. Die das Faserband bildenden Fasern oder Filamente sind üblicherweise gekräuselt, vorzugsweise zwei- oder dreidimensional gekräuselt. Der Kabeltiter beträgt zweckmäßigerweise etwa 1 bis 12 ktex, vorzugsweise 4 bis 12 ktex, insbesondere 6 bis 8 ktex. Der Kabeltiter wird unter anderem durch die Dimensionierung der Verarbeitungsanlage vorgegeben, beispielsweise durch die Auslegung der Transporteinrichtungen und/oder durch die Dimensionierung der Erhitzungs- oder Abkühlzone. Es können also auch ohne weiteres andere Kabeltiter als die oben genannten zum Einsatz kommen.

Der Einzeltiter der das Faserkabel bildenden Fasern oder Filamente liegt üblicherweise zwischen 2 bis 15 dtex, vorzugsweise zwischen 4 und 7 dtex. Es können auch Titermischungen eingesetzt werden. Die Einzeltiter der Füllfasern und der Bindefasern werden so ausgewählt, daß bei vorgegebener Heizleistung in der Erhitzungszone und bei vorgegebener Transportgeschwindigkeit des Kabels durch diese Zone zumindest auf der Oberfläche des Faserkabels ein so weitgehendes Verkleben beider Fasersorten erfolgen kann, daß in der nachfolgenden Abkühlzone eine für den ins Auge gefaßten Verwendungszweck ausreichende Verfestigung erzielt werden kann.

Als Füllfasern im Faserkabel können im Prinzip alle für diesen Zweck geeigneten Textilfasern verwendet werden. Dabei kann es sich um Naturfasern handeln, beispielsweise um Baumwolle oder Schafwolle, oder es können Chemiefasern, insbesondere Synthesefasern verwendet werden, beispielsweise Polyamid-, Polyester- oder Polyacrylnitril-Fasern. Bevorzugt setzt man als Füllfasern Polyesterfasern, insbesondere Fasern auf Basis von Polyethylenterephthalat ein.

Als Bindefasern im Faserkabel können im Prinzip alle Fasern aus thermoplastischem Material verwendet werden, wobei im Falle von thermoplastischen Füllfasern Bindefasern mit einem tieferen Erweichungspunkt als die Füllfasern eingesetzt werden müssen, vorzugsweise Bindefasern mit einem um 10°C, insbesondere mit einem um 30°C tiefer liegenden Erweichungspunkt als die thermoplastischen Füllfasern.

Bevorzugt verwendet man als Bindefasern Fasern aus thermoplastischem Polyester, bevorzugt auf der Basis von Polyethylenterephthalat. insbesondere auf der Basis von modifiziertem Polyethylenterephthalat. Ganz besonders bevorzugt verwendet man Bindefasern aus Polyester Bikomponentenfasern mit Kern/Mantel Struktur, wobei der Mantel insbesondere als Copolyester ausgebildet ist, der einen tieferen Erweichungspunkt besitzt als der den Kern bildende Polyester. Es lassen sich aber auch Bikomponentenfasern verwenden, die aus unterschiedlichen Polymeren bestehen, beispielsweise aus einem Kern aus Polyester und einem Mantel aus einem niedriger schmelzenden Polymeren, z.B. einem Polyolefin. Beispiele für geeignete Bikomponentenfasern sind in der US-A-3,589,956 beschrieben.

Die Füllfasern können bereits bei der Herstellung des Faserkabels mit den Bindefasern kombiniert werden oder es werden Faserkabel, insbesondere Kardenbänder oder Krempellunten, aus verschiedenen Fasersorten vor der Erhitzungszone miteinander kombiniert und dieser Erhitzungszone gemeinsam zugeführt. Dieses Kombinieren kann durch einfaches Zusammenführen der Faserkabel erfolgen oder durch Doublieren verschiedener Faserkabel. Der Anteil der Füllfasern im Faserkabel beträgt etwa 50 - 95 Gew.-%, bezogen auf die Menge an Füllfasern und Bindefasern und der Anteil der Bindefasern beträgt entsprechend 50 - 5 Gew.-%. Vorzugsweise beträgt der Anteil der Füllfasern etwa 75 Gew.-% und der Anteil der Bindefasern beträgt etwa 25 Gew.-%.

Die Temperatur in der Erhitzungszone ist so zu wählen, daß die Bindefasern zumindest auf der Oberfläche des Faserkabels mit den Füllfasern verkleben, so daß sich das Kabel in der nachfolgenden Abkühlzone so weit verfestigt, daß eine für die Verwendung als Füllmaterial ausreichende Stabilität erreicht wird. Die Bindefasern können selbstverständlich in der Erhitzungszone im gesamten Volumen des Faserkabels aufgeschmolzen werden.

Das Erhitzen kann in jeder beliebigen Art und Weise erfolgen, beispielsweise durch Strahlung oder durch Kontakt mit heißen Fluiden oder mit erhitzten Führungselementen für das Faserkabel. So läßt sich das Faserkabel z.B. durch eine Erhitzungszone leiten, in der eine Infrarotstrahlung oder eine Mikrowellenstrahlung auf das Faserkabel einwirkt, oder in der ein erhitztes Fluid, insbesondere erhitzte Luft, durch das Faserkabel gepreßt und/oder gesaugt wird oder über dessen Oberfläche geleitet wird, oder das Faserkabel wird bei der Führung durch ein Rohr durch Kontakt mit den erhitzten Innenseiten dieses Rohres zumindest oberflächig aufgeschmolzen.

In der Abkühlungszone kann das erhitzte Kabel entweder durch ein Kühlaggregat transportiert werden oder man verwendet die Umgebungstemperatur zum Abkühlen. In jedem Fall muß das Faserkabel in dieser Zone soweit abgekühlt werden, daß es fur die nachfolgende Schneideoperation eine genügende Festigkeit aufweist und beim Zerschneiden nicht in Einzelfasern zerfällt.

Verwendet man ein Kühlaggregat, so eignen sich als Kühlmittel insbesondere gekühlte Fluide, insbesondere gekühle Luft, die durch das Faserkabel gepreßt und/oder gesaugt werden oder über dessen Oberfläche geleitet werden oder das Faserkabel wird bei der Führung durch ein Rohr durch Kontakt mit den gekühlten Innenflächen dieses Rohres zumindest oberflächig soweit abgekühlt, daß die gewünschte Verfestigung erzielt wird.

Das Zerschneiden des verfestigten Faserkabels kann mit jeder der für Faserkabel bekannten Schneidevorrichtungen durchgeführt werden. Beispiele dafür sind Schneideräder mit nach außen feststehenden Messern, auf die das verfestigte Kabel aufgewickelt wird, bis der durch die Wicklung entstehende Druck auf die Innenlagen des Kabels so groß wird, daß diese Innenlagen durch die Messer zerschnitten werden. Das Kabel kann auch an einem rotierenden Messerkopf vorbeigeführt werden, wobei die Messer senkrecht zur Transportrichtung des Kabels rotieren und diese zerschneiden.

Vorzugsweise führt man das Kabel unter Andruck unter oder über einem rotierenden Schneiderad vorbei, so daß das Kabel nicht aufgewickelt zu werden braucht. Zum Zerschneiden verwendet man Schneidevorrichtungen mit erhitzten Messern, so daß während des Schneidevorgangs an der entstehenden Schnittfläche eine solche Temperatur herrscht, daß die die Bindefasern zumindest an der Oberfläche der entstehenden Schnittfläche mit den Füllfasern verkleben, so daß hier eine im wesentlichen glatte Oberfläche entsteht, die praktisch keine freien Faserenden aufweist. Auf diese Weise lassen sich rechteckige oder quadratische Flocken erzeugen.

Der Transport des Faserkabels durch die unterschiedlichen Verarbeitungszonen wird durch dafür übliche Transportvorrichtungen durchgeführt. Düsen, durch die das Faserkabel mittels eines Transportmediums gefördert wird oder insbesondere Rollenpaare, die das Faserkabel an einer oder mehreren Stellen der Vorrichtung unterstützen und transportieren. Vorzugsweise befinden sich solche Transportrollen zwischen der Abkühlzone und der Schneidevorrichtung. Dabei kann eine Rolle den Transport bewerkstelligen und die andere Rolle bewirkt den Andruck des Faserkabels an die Transportrolle. Die Erfindung betrifft auch eine Vorrichtung zur Durchführung des beanspruchten Verfahrens, wie dies in Anspruch 10 definiert ist. Besonders bevorzugte Ausführungen des erfindungsgemäßen Verfahrens bzw. der Vorrichtung sind in den Ansprüchen 2 bis 9 bzw. 11 bis 13 definiert. Im folgenden werden das Verfahren und die dazu geeignete Vorrichtung anhand von Zeichnungen beispielhaft beschrieben.

Figur 1 zeigt schematisch die Seitenansicht einer Vorrichtung zur Verarbeitung eines Kardenbandes oder einer Krempellunte nach dem erfindungsgemäßen Verfahren.

Figur 2 zeigt die Seitenansicht einer Ausgestaltung der Erhitzungs- und Kühlvorrichtung der erfindungsgemäßen Vorrichtung.

Figur 3 zeigt einen Längsschnitt durch eine Erhitzungsvorrichtung der erfindungsgemäßen Vorrichtung.

Figur 4 zeigt einen Längsschnitt durch eine Kühlvorrichtung der erfindungsgemäßen Vorrichtung.

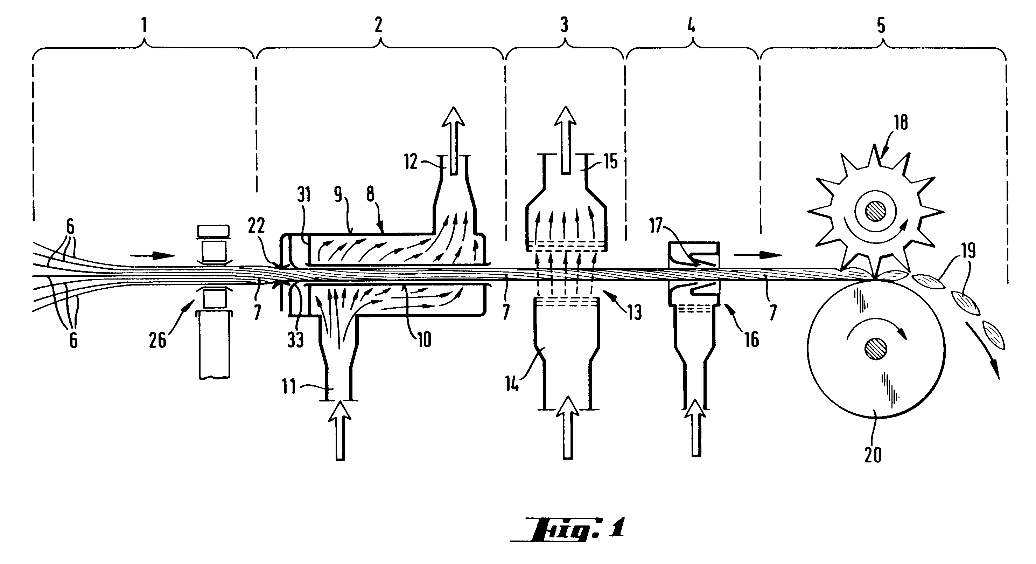

In Figur 1 ist schematisch eine Ausführungsform des beanspruchten Verfahrens dargestellt. Die von der Krempel kommende Krempellunte (6) wird über eine Zuführzone (1) einer Erhitzungszone (2) zugeführt, an die sich eine Abkühlungssone (3), eine Transportzone (4) und eine Schneidezone (5) anschließt. Die Krempellunte (6) wird vor dem Eintritt in die Heizvorrichtung (8) durch Aufbringen eines Falschdralls mittels der Vorrichtung (26) noch mechanisch stabilisiert. Dieser Verfahrensschritt ist nicht zwingend, jedoch insbesondere bei Verwendung von Kardenbändern oder Krempellunten aus Fasern bevorzugt. Die Stabilisierung kann in an sich bekannter Weise erfolgen, beispielsweise durch Falschdrallen der Krempellunte (6) mit Luft oder mittels mechanischer Reibung, beispielsweise durch Nitscheln.

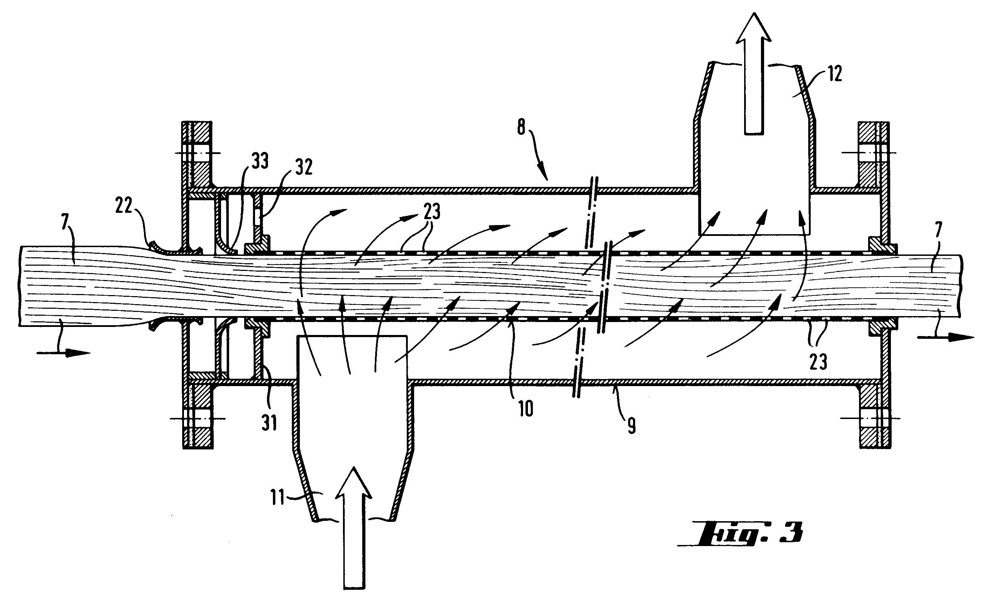

Nach dem Aufbringen des Falschdralls liegt ein mechanisch verfestigtes Faserkabel (7) vor, das in eine Heizvorrichtung (8) eingeführt wird. In Figur 1 ist diese Heizvorrichtung als Mantelheizung dargestellt, wobei das Kabel (7) in einem Rohr (10) geführt wird. Dieses Rohr (10) ist von einem Heizmantel (9) umgeben, durch den ein Fluid strömt, welches dem Mantel (9) über den Eintrittstutzen (11) zugeführt wird und über den Austrittstutzen (12) abgeführt wird. Eintrittstutzen (11) und Austrittstutzen (12) können auch gegensinnig angeordnet sein. Das Rohr (10) kann von dem Fluid umströmt werden, so daß das Erhitzen des Kabels (7) wie dargestellt über den Kontakt mit den heißen Rohrwänden erfolgt, oder das Rohr (10) kann Öffnungen aufweisen, so daß das Fluid selbst mit dem Kabel (7) in Kontakt treten kann. Vorzugsweise verwendet man als Fluid erhitzte Luft und läßt diese durch ein durchlöchertes Rohr (10) direkt auf das Kabel (7) einwirken (hier nicht dargestellt; vergl. Figur 3). Durch Auswahl eines engeren Rohrdurchmessers kann das Kabel (7) zusätzlich kompaktiert werden. Dabei wird das Einführen des Kabels (7) in das Rohr (10) durch Düsen (22) und (33) begünstigt. An die Erhitzungszone (2) schließt sich die Abkühlungszone (3) an. In der Ausführungsform von Figur 1 ist diese als Abkühlungsaggregat (13) dargestellt. Dazu wird ein als Kühlmittel wirkendes Fluid, vorzugsweise Luft, mit dem Faserkabel entweder direkt oder über eine Kühlfläche (nicht dargestellt) in Kontakt gebracht. Das Fluid kann in oder gegen die Kabeltransportrichtung oder, wie dargestellt, vorzugsweise senkrecht dazu strömen. In Fig. 1 wird das Fluid durch den Eintrittstutzen (14) zugeführt, durch das Kabel (7) gedrückt und über den Austrittstutzen (15) abgeführt. Das durch Abkühlen verfestigte Kabel (7) wird anschließend einer Transportvorrichtung (16) zugeführt, die in der in Figur 1 dargestellten Form eine Ringdüse (17) ist, in der das Kabel (7) mittels eines Fluids, vorzugsweise Luft, bewegt wird. Nach Passieren der Transportzone (4) wird das verfestigte Kabel einer Schneidezone (5) zugeführt, in der es durch ein Schneiderad (18) in Flocken (19) zerschnitten wird. Das Kabel (7) wird an das Schneiderad (18) durch eine Andruckwalze (20) angepresst.

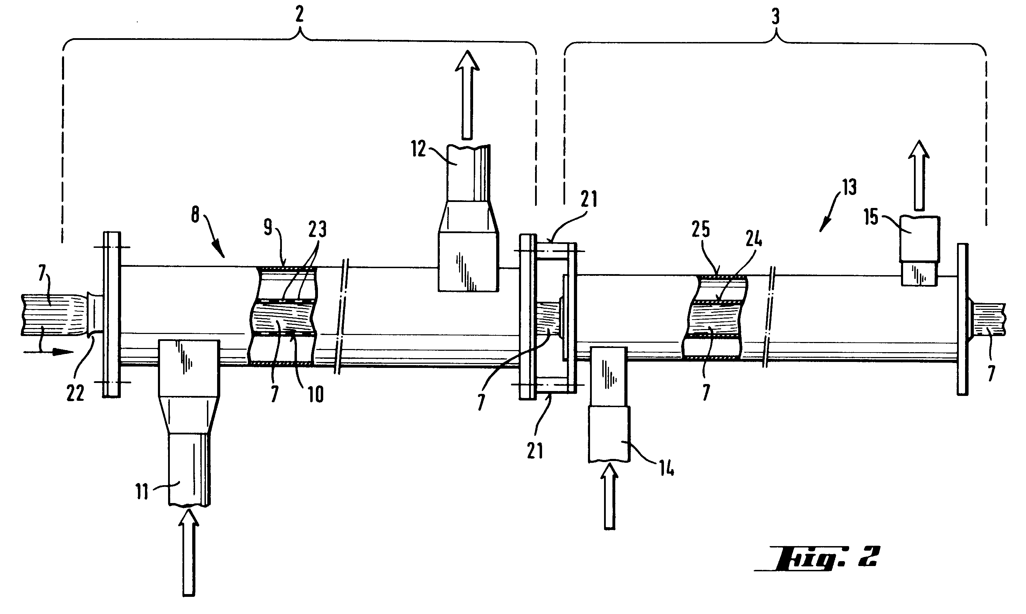

In Figur 2 ist eine besondere Ausgestaltung der Erhitzungszone (2) und der Abkühlzone (3) dargestellt. Die Heizvorrichtung (8) ist hier ebenfalls als Mantelheizung ausgeführt. Der Heizmantel (9) wird über den Eintrittstutzen (11) und den Austrittstutzen (12) mit dem Heizfluid versorgt. Das Faserkabel (7) wird durch die Eintrittsdüse (22) in das Führungsrohr (10) und dort vorzugsweise kompaktiert eingeführt. Die Heizvorrichtung (8) ist über Distanzbüchsen (21) direkt mit dem Abkühlungsaggregat (13) verbunden, welches von einem Kühlmantel (25) umgeben ist. Das Kühlmittel wird dem Kühlmantel über den Eintrittstutzen (14) zugeführt und durch den Austrittstutzen (15) abgeführt.

Figur 3 zeigt eine besondere Ausführungsform der Heizvorrichtung (8). In dieser Ausführungsform ist das Rohr (10) mit Löchern (23) versehen, so daß das durch den Mantel (9) geführte Fluid durch das Rohr (10) und das darin geführte Kabel (7) strömen kann. Das Rohr (10) ist vorzugsweise auf seiner ganzen Länge durchlöchert. Es sind aber auch Ausführungsformen möglich, in denen nur ein Teil des Rohres (10) durchlöchert ist. Die Halterung (31) für das Führungsrohr (10) ist mit Öffnungen (32) für das Heizfluid versehen. Durch die trichterartige Gestaltung des Einlasses (33) wird so der Einzug des Faserkabels (7) nochmals verbessert.

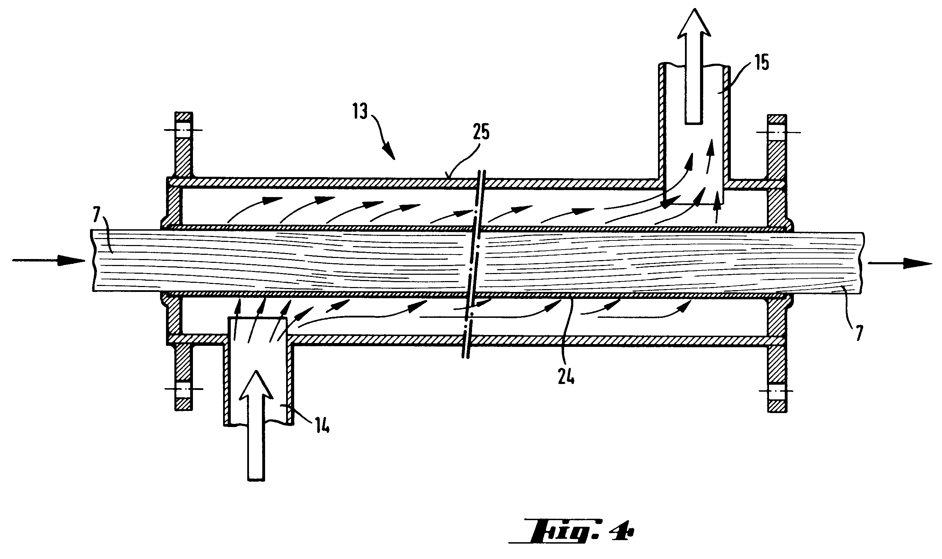

In Figur 4 ist das Abkühlungsaggregat (13) ausführlicher und geschnitten dargestellt. Im Innern verläuft ein Rohr (24), in dem das Kabel (7) geführt wird. Dieses Rohr (24) kann wie bei der Heizvorrichtung durchlöchert sein. Im Falle von flüssigen Abkühlmedien ist das Rohr (24) jedoch vorzugsweise geschlossen und das Abkühlmedium wirkt in diesem Falle nur indirekt über die Rohrwände auf das Kabel (7) ein. Das Rohr (24) ist vom Mantel (25) umgeben, der über den Eintrittstutzen (14) und den Austrittstutzen (15) mit dem Kühlmittel versorgt wird, wobei die Stutzen (14) und (15) auch gegensinnig angeordnet sein können.

Das folgende Beispiel erläutert die Erfindung ohne diese zu begrenzen.

Über Krempeln werden Fasermischungen aus Polyethylenterephthalat und Bikomponentenfasern auf Basis von Polyethylenterephthalat und modifiziertem Polyethylenterephthalat (Kern/Mantel Fasern) zu Krempellunten zusammengefaßt. Man verwendet als Füllfasern TREVIRA^{(R)} 290 (Stapelfaser 6,7 dtex/60 mm) oder TREVIRA^{(R)} 206 (Stapelfaser 6,0 dtex/60 mm) und als Bindefasern TREVIRA^{(R)} 252 (Stapelfaser 3,0 dtex/50 mm) in einer Mischung von 75 Gew.-% Füllfaser und 25 Gew.-% Bindefaser. Die Krempellunten werden durch Wärmeeinwirkung verfestigt. Die Temperatur beträgt etwa 180°C. Anschließend erfolgt sofort eine Abkühlung des thermisch behandelten Materials, wobei es vorzugsweise geformt wird. Danach werden die geformten und verfestigten Lunten in unterschiedliche Schnittlängen, bevorzugt 2 - 6 cm geschnitten und in Bettwarenartikel, wie Kissen, geblasen. Das Material neigt nicht zur Klumpenbildung und damit gefüllte Kissen lassen sich aufschütteln, ohne daß die Flocken zerfallen oder sich verhaken.

## Patentansprüche

1. Verfahren zur Herstellung von Füllmaterial ausgehend von einem Faserkabel aus Endlosfasern oder aus Stapelfasern enthaltend mindestens eine Art von Füllfasern und mindestens eine Art von Bindefasern umfassend die Schritte:
a) kontinuierliches Führen des Faserkabels durch eine Erhitzungszone und Erhitzen des Faserkabels auf eine Temperatur, bei der die Bindefasern zumindest auf der Oberfläche des Faserkabels mit den Füllfasern verkleben,
b) kontinuierliches Führen des Faserkabels durch eine Abkühlungszone, wobei die Temperatur des Faserkabels so weit abgesenkt wird, daß ein zumindest an der Oberfläche verfestigtes Faserkabel entsteht, und
c) kontinuierliches Zuführen des verfestigten Faserkabels zu einer Schneidevorrichtung und Zerschneiden des Faserkabels mit einer erhitzten Schneidevorrichtung in Stücke, so daß die Bindefasern an den Schnittflächen mit den Füllfasern verkleben und auf den Schnittflächen eine im wesentlichen glatte Oberfläche entsteht, insbesondere in Stücke mit einer Schnittlänge von 2 bis 6 cm.

2. Verfahren gemäß Anspruch 1, dadurch gekennzeichnet, daß das Faserkabel aus Stapelfasern besteht, die insbesondere in Form einer Krempellunte vorliegen.

3. Verfahren gemäß Anspruch 2, dadurch gekennzeichnet, daß die Krempellunte vor Schritt a) durch Aufbringen eines Falschdralls mechanisch stabilisiert wird.

4. Verfahren gemäß einem der Ansprüche 2 oder 3, dadurch gekennzeichnet, daß die Krempellunte in der Erhitzungs- und in der Kühlzone so geführt wird, daß besagte Krempellunte kompaktiert und verfestigt wird.

5. Verfahren gemäß einem der Ansprüche 1 bis 4, dadurch gekennzeichnet, daß die Füllfasern aus Polyester, insbesondere aus Polyethylenterephthalat, bestehen.

6. Verfahren gemäß Anspruch 5, dadurch gekennzeichnet, daß die Bindefasern aus Polyester, insbesondere aus einem modifiziertem Polyethylenterephthalat, bestehen.

7. Verfahren gemäß Anspruch 6, dadurch gekennzeichnet, daß das Faserkabel einen Titer zwischen 1 und 12 ktex aufweist, der Einzeltiter der Füll- und der Bindefasern zwischen 2 und 15 dtex liegt, der Gewichtsanteil der Füllfasern 50 bis 95 % und derjenige der Bindefasern 50 bis 5 % ausmacht, wobei die Füllfasern zwei- oder dreidimensional gekräuselte Polyesterfasern, insbesondere Polyethylenterephthalatfasern sind, und die Bindefasern aus Polyesterfasern bestehen, vorzugsweise aus Fasern aus einem modifizierten Polyethylenterephthalat, deren Erweichungspunkt mehr als 10°C unter dem Erweichungspunkt der Füllfasern liegt.

8. Verfahren gemäß Anspruch 7, dadurch gekennzeichnet, daß die Bindefasern aus Polyester Bikomponentenfasern mit Kern/Mantel Struktur bestehen, wobei der Mantel insbesondere als Copolyester ausgebildet ist, der einen tieferen Erweichungspunkt besitzt als der den Kern bildende Polyester.

9. Verfahren gemäß einem der Ansprüche 1 bis 8, dadurch gekennzeichnet, daß die erhaltenen Flocken direkt nach Schritt c) in zu füllendes dreidimensionales textiles Gebilde eingebracht, insbesondere eingesaugt oder eingeblasen werden.

10. Vorrichtung zur Durchführung des Verfahrens nach einem der Ansprüche 1 bis 9 umfassend eine Fertigungsstrecke, in der das Faserkabel kontinuierlich von einer Zuführzone (1) durch eine Heizvorrichtung (8) und durch eine Abkühlvorrichtung (13) einer beheizbaren Schneidevorrichtung (18) zugeführt wird, wobei der Transport des Faserkabels durch eine Transportvorrichtung (16) erfolgt, die sich zwischen der Abkühlvorrichtung (13) und der Schneidevorrichtung (18) befindet.

11. Vorrichtung gemäß Anspruch 10, dadurch gekennzeichnet, daß sich in der Zuführzone (1) eine Vorrichtung (26) befindet, mit der einer Krempellunte ein Falschdrall aufgeprägt werden kann.

12. Vorrichtung gemäß einem der Ansprüche 10 oder 11, dadurch gekennzeichnet, daß die Heizvorrichtung als Mantelheizung ausgeführt ist, bei der ein Führungsrohr (10) für das Faserkabel mit durch Löcher (23) durchbrochenen Seitenwänden in einem Heizmantel (9) verläuft, der Zuleitungen (11) und Ableitungen (12) für das Erhitzungsmedium, vorzugsweise erhitzte Luft, aufweist und wobei das Führungsrohr (10) einen geringeren Durchmesser als das in den Einzugstrichter (22) eintretende Faserkabel (7) besitzt, so daß besagtes Faserkabel im Führungsrohr (10) kompaktiert und mechanisch stabilisiert wird.

13. Vorrichtung gemäß einem der Ansprüche 10 bis 12, dadurch gekennzeichnet, daß die Abkühlvorrichtung aus einem Mantelrohr (25) besteht, durch dessen Ringraum das Kühlmedium, vorzugsweise Wasser geschickt wird und das ein nicht durchbrochenes Innenrohr (24) aufweist, durch das das verfestigte Faserkabel (7) verläuft.

## Claims

1. A process for producing filling material starting from a tow of continuous filament or staple fiber containing at least one kind of filling fiber and at least one kind of binding fiber, comprising the steps of:
a) continuously guiding the tow through a heating zone and heating the tow to a temperature at which the binding fibers become fused to the filling fibers at least at the surface of the tow,
b) continuously guiding the tow through a cooling zone wherein the temperature of the tow is lowered to such an extent as to form a tow which is consolidated at least at the surface, and
c) continuously guiding the consolidated tow to a cutting means and cutting the tow into pieces with a heated cutting means so that the binding fibers become fused to the filling fibers at the surfaces created by the cutting and an essentially smooth surface forms on the surfaces created by the cutting, in particular into pieces from 2 to 6 cm in length.

2. The process of claim 1, wherein the tow consists of staple fibers which are in particular in the form of a roller-and-clearer card sliver.

3. The process of claim 2, wherein the roller-and-clearer card sliver is mechanically stabilized by application of a false twist prior to step a).

4. The process of either of claims 2 and 3, wherein the roller-and-clearer card sliver is guided within the heating and cooling zones in such a way that said sliver becomes compacted and consolidated.

5. The process of any one of claims 1 to 4, wherein the filling fibers are made of polyester, in particular polyethylene terephthalate.

6. The process of claim 5, wherein the binding fibers are made of polyester, in particular a modified polyethylene terephthalate.

7. The process of claim 6, wherein the tow has a linear density between 1 and 12 ktex, the filling and binding fibers have a linear density between 2 and 15 dtex, and the weight portion of filling fiber is from 50 to 95% and that of binding fiber is from 50 to 5%, the filling fiber being two- or three-dimensionally crimped polyester fiber, in particular polyethylene terephthalate fiber, and the binding fiber comprising polyester fiber, preferably fiber made of a modified polyethylene terephthalate whose softening point is more than 10°C below the softening point of the filling fiber.

8. The process of claim 7, wherein the binding fiber comprises a polyester bicomponent fiber having a core/sheath structure in which the sheath is in particular constituted by a copolyester which has a lower softening point than the polyester which forms the core.

9. The process of any one of claims 1 to 8, wherein, immediately following step c), the tufts obtained are introduced into a three-dimensional textile structure to be filled, in particular by being sucked or blown into it.

10. Apparatus for carrying out the process of any one of claims 1 to 9, comprising a production line in which the tow is continuously guided from a feed zone (1) through a heating means (8) and through a cooling means (13) toward a heatable cutting means (18), the transport of the tow being effected by a transport means (16) which is situated between the cooling means (13) and the cutting means (18).

11. Apparatus as claimed in claim 10, wherein the feed zone (1) contains a device (26) whereby a false twist can be applied to a roller-and-clearer card sliver.

12. Apparatus as claimed in either of claims 10 and 11, wherein the heating means is constructed as a jacket heater in which a tow guide tube (10) which has holes (23) in the side walls extends within a heating jacket (9) which has inlet lines (11) and outlet lines (12) for the heating medium, preferably hot air, the guide tube (10) having a smaller diameter than the tow (7) entering the intake funnel (22), so that said tow is compacted and mechanically stabilized within the guide tube (10).

13. Apparatus as claimed in any one of claims 10 to 12, wherein the cooling means comprises a jacketed tube (25) whose annular space is charged with the cooling medium, preferably water, and which has a non-perforated inner tube (24) through which the consolidated tow (7) runs.

## Revendications

1. Procédé pour la préparation de matière de remplissage en partant d'un câble de fibres à filaments continu ou de fibres coupées contenant au moins un type de fibres de remplissage et au moins un type de fibres de liaison comprenant les étapes :
a) conduite en continu du câble de fibres travers une zone de réchauffage et réchauffage du câble de fibres à une température à laquelle les fibres de liaison doivent coller au moins à la surface du câble de fibres avec les fibres de remplissage;
b) conduite en continu du câble de fibres à travers une zone de refroidissement, la température du câble de fibres ayant alors été réduite dans une telle mesure qu'il se forme au moins un câble de fibres consolidé, et
c) admission en continu du câble de fibres compacté vers un dispositif de découpage et découpe du câble de fibres en morceaux à l'aide d'un dispositif de découpage réchauffé de sorte que les fibres de liaison collent aux fibres de remplissage sur les surfaces de coupe et il se forme sur les surface de coupe une surface essentiellement lisse, notamment en morceaux avec une longueur de découpe de 2 à 6 cm.

2. Procédé selon la revendication 1, caractérsié en ce que le câble de fibres est constitué de fibres coupées qui sont présentes sous une forme particulière d'une mêche cardée.

3. Procédé selon la revendication 2, caractérisé en ce que la mêche cardée est stabilisée mécaniquement avant l'étape a) par application d'une fausse torsion.

4. Procédé selon l'une des revendication 2 ou 3, caractérisé en ce que la mêche cardée est conduite à travers la zone de réchauffage et la zone de refroidissement de telle façon que ladite mêche cardée est compactée et consolidée.

5. Procédé selon l'une des revendications 1 à 4, caractérisé en ce que les fibres de remplissage sont constituées de polyester, plus particulièrement de téréphtalate de polyéthylène.

6. Procédé selon la revendication 5, caractérisé en ce que les fibres de liaison sont constituées de polyester, plus particulièrement d'un téréphtalate de polyéthylène modifié.

7. Procédé selon la revendication 6, caractérisé en ce que le câble de fibres présente un titre entre 1 et 12 ktex, le titre unitaire des fibres de remplissage et des fibres de liaison entre 2 et 15 dtex, la part pondérale des fibres de remplissage est de 50 à 95% et celle des fibres de liaison de 50 à 5%, les fibres de remplissage étant des fibres polyester ondulées bidimensionnellement ou tridimensionnellement, notamment les fibres de téréphtalate de polyéthylène, et les fibres de liaison sont constituées de fibres de polyester, de préférence de fibres d'un téréphtalate de polyéthylène modifié, dont le point de ramollissement est situé à plus de 10°C au-dessous du point de ramollissement des fibres de remplissage.

8. Procédé selon la revendication 7, caractérisé en ce que les fibres de liaison sont constituées de fibres polyester bicomposantes avec une structure âme/gaine, la gaine étant formée notamment en tant que copolyester qui a un point de ramollissement inférieur à celui du polyester formant l'âme.

9. Procédé selon l'une des revendications 1 à 8, caractérisé en ce que les flocons obtenus sont incorporés immédiatement après l'étape c) dans le produit textile tridimensionnel à remplir, notamment aspirés ou insufflés.

10. Dispositif pour la mise en oeuvre du procédé selon l'une des revendications 1 à 9, comprenant une chaîne de production dans laquelle est conduit en continu le câble de fibres d'une zone d'alimentation (1) à travers un dispositif de chauffage (8) et à travers un dispositif de refroidissement (13) d'un dispositif de découpage (18) chauffable, le transport du câble de fibres étant effectué à l'aide d'une installation de transport (16) qui se trouve entre le dispositif de refroidissement (13) et le dispositif de découpage (18).

11. Dispositif selon la revendication 10, caractérisé en ce que dans la zone d'admission (1) se trouve un dispositif (26) à l'aide duquel on peut appliquer par gaufrage une fausse torsion à la mêche cardée.

12. Dispositif selon l'une des revendications 10 ou 11, caractérisé en ce que le dispositif de chauffage est réalisé en tant que chauffage de la chemise, dans laquelle un tube-guide (10) pour le câble de fibres passe à travers les parois latérales perforées de trous (23) dans une chemise chauffante (9), qui présente des conduites d'admission (11) et d'évacuation (12) pour le milieu de chauffage, de préférence l'air réchauffé, et le tube-guide (10) ayant un diamètre inférieur à celui du câble de fibres (7) entrant dans la trémie d'entrée (22), de sorte que ledit câble de fibres est compacté et stabilisé mécaniquement dans le tube-guide (10).

13. Dispositif selon l'une des revendications 10 à 12, caractérisé en ce que le dispositif de refroidissement est constitué d'un tube de protection (25), à travers l'espace annulaire duquel est envoyé le milieu réfrigérant, de préférence l'eau, et qu'il présente une tube intérieur non perforé (24), à travers lequel passe le câble de fibres (7) consolidé.
